# EUROPEAN PATENT SPECIFICATION

(11) **EP 2 692 382 B1**
(45) Date of publication and mention of the grant of the patent: **05.04.2017**
(21) Application number: 12763082.0
(22) Date of filing: 24.01.2012
(51) Int. Cl.: A61M 16/16, A61M 11/06, A61M 16/10, A61M 16/14

(54) **NEBULIZER SYSTEM AND HEATER DEVICE USED IN SAID NEBULIZER SYSTEM**
VERNEBLERSYSTEM UND IN DIESEM VERNEBLERSYSTEM VERWENDETE HEIZVORRICHTUNG
SYSTÈME DE NÉBULISEUR ET DISPOSITIF DE CHAUFFAGE UTILISÉ DANS LEDIT SYSTÈME DE NÉBULISEUR

(30) Priority: 31.03.2011 JP 2011079391
(43) Date of publication of application: 05.02.2014
(73) Proprietor: Japan Medicalnext Co., Ltd., Osaka-shi, Osaka 541-0042 (JP)
(72) Inventor: Yuki Ken, Osaka-shi, Osaka 541-0042 (JP); Haramiishi Yoshihisa, Osaka-shi, Osaka 541-0042 (JP); Shimotoso Toshihiko, Osaka-shi, Osaka 541-0042 (JP)
(74) Representative: Horn Kleimann Waitzhofer Patentanwälte PartG mbB
(86) International application number: PCT/JP2012/051396
(87) International publication number: WO 2012/132517

(56) References cited:
- JP-A- S 581 463
- JP-A- 54 127 193
- JP-A- 58 001 463
- JP-A- 2010 119 838
- US-A- 3 990 441
- US-A- 4 101 611
- US-A- 4 190 046
- US-A- 4 427 004
- US-A- 4 911 157
- US-A- 5 259 370

## Description

### TECHNICAL FIELD

The present invention relates to a nebulizer system that adds moisture (water vapor or water mist) to gas such as air or oxygen, and to a heater device for use in the nebulizer system.

### BACKGROUND ART

Heretofore, to treat patients with respiratory system, there has been used oxygen therapy for supplying oxygen to the patients. In the therapy, oxygen delivered from, for example an oxygen cylinder, or oxygen including absorbents such as molecular sieves and concentrated by an oxygen concentrator is supplied to the patients by means of a nose cannula, respiratory mask, or the like. Since oxygen supplied from an oxygen cylinder, or the like contains little moisture, when oxygen is supplied into a patient's respiratory tract including a nasal cavity, it is necessary to prevent inside the respiratory tract from drying. For this reason, a humidifier is provided in the middle of an oxygen supply tube to supply humidified oxygen.

In oxygen therapies, a nebulizer system has been known as the humidifier used for humidifying oxygen (refer to e.g. Patent Document 1). As illustrated in FIG. 18, the nebulizer system includes a bottle (container) 101 containing a solution, in which medication is dissolved, or liquid such as sterile water, purified water, distilled water, and saline solution, and a nebulizer adapter 110 that is connected to the bottle 101. The nebulizer system 100 is a humidifier configured such that gas such as oxygen gas is supplied into the adapter 110, thereby sucking up sterile water contained in the bottle 101 while taking air therein, and humidifying gas with high oxygen concentration by the sucked sterile water as fine aerosol to supply the humidified gas to a patient. Also, in order to return the liquid that is not aerosolized in the adapter 110 to the bottle (container) 101, the nebulizer system 100 includes a drain tube 105 that connects the nebulizer adapter 110 with the bottle 101. In some cases, the nebulizer system may employ a configuration, in which a heater device is interposed between the bottle (container) and the adapter, thereby sucking up the sterile water contained in the bottle while heating the sterile water, and humidifying gas with high oxygen concentration by sucked and heated sterile water as fine aerosol to supply the humidified gas to a patient.

### PRIOR ART DOCUMENT

### PATENT DOCUMENT

Patent Document 1: JP 2004-141493 A

The above-described nebulizer system includes a drain tube that connects the nebulizer adapter with the bottle as a feature for returning droplets collected in the nebulizer adapter to the bottle. However, the drain tube that is external to the bottle may be caught by a person's hand or thing, which would result in toppling of the nebulizer system. Further, since the drain tube is fixedly connected to the nebulizer adapter, if the nebulizer adapter is screw-connected to the heater device and to the bottle, the drain tube gets in the way, leading to a drawback that handling of connection work is quite inferior.

Also, the drain tube requires to be connected to the bottle after the nebulizer adapter is connected to the bottle and heater device, leading to the drawback that additional time is required for setting the nebulizer adapter. Further, at the time when the nebulizer adapter is connected to the heater device and bottle, the drain tube may contact with various objects in the patient's room (such as a human body, desk, bed, and wall). If such a contact occurs, the tip of the drain tube that must be kept clean would be contaminated. If such a contaminated drain tube is connected to the bottle, liquid in the bottle would also be contaminated, which has been another drawback.

US Patent 4,427,004 discloses a linear, annular flow nebulizer for use in inhalation therapy comprising a shell having walls with an ambient air intake in one of the walls; a nebulizer conduit having a proximal end, a distal end and a longitudinal fluid passageway, the proximal end communicating with a gas source and the distal end inwardly tapering to define a nozzle which penetrates a wall of the shell; and, a nebulizer cup having a bottom end mounted within the interior space of the shell, the bottom of the cup having a well surrounding a dispensing port spaced from and in alignment with the nozzle, the well permitting the angular confluence of a liquid stream into a gas stream to produce an aerosol.

US Patent 4,911,157 discloses a nebulizer system for producing a humidified and heated breathing gas to be inhaled by a patient undergoing inhalation therapy. The nebulizer system includes a nebulizer module and a heater module affixed to one another and optionally detachably connected to each other. The nebulizer module is connectable to an oxygen source, a liquid water supply, and, through an outlet, to a breathing apparatus. A throat in the system serves to direct an aerosol spray produced in the nebulizer module to a heat transfer element in a conical flow pattern.

US Patent 5,259,370 discloses a nebulizer providing a moistened breathing mixture of aerosol for inhalation therapy is provided with an improved accumulator chamber and heater configured to heat aerosol discharged from the nebulizer so as to most efficiently utilize the heat energy and to most efficiently transfer heat energy from the heater to the aerosol. Passage of the aerosol through and accumulation in a relatively long annular accumulator chamber that adjoins the heater allows introduction of heated water vapor into the aerosol.

JP S58-1463 discloses another nebulizer with a heater, in which the heater is disposed inside a vessel.

US Patent 4,101,611 discloses a nebulizer comprising a container with a supply of water, a downwardly opening cap with air delivery means and housing an aspirator connected with a supply of oxygen under pressure and adapted to draw water from said supply, an elongate heating unit with an upper end defining a heating chamber and carried by the container, said cap engaged with the upper end of the unit to define a mixing chamber of fixed predetermined volume and shape in which the aspirator is positioned, water inlet means communicating with the heating chamber and water supply, water outlet means communicating with the heating chamber and the aspirator and an electric resistance heater in the unit, spaced from the heating chamber to heat water in the heating chamber and temper the atmosphere in the mixing chamber.

### SUMMARY OF THE INVENTION

### PROBLEMS TO BE SOLVED BY THE INVENTION

Consequently, it is an object of the present invention to provide a nebulizer system that allows an easy connection of a bottle, a heater device, and a nebulizer adapter, while eliminating a drain tube that is exposed to the outside to get in the way, and a heater device preferably for use in the nebulizer system.

### MEANS FOR SOLVING THE PROBLEMS

The above described object of the present invention is achieved by a nebulizer system having the features recited in claim 1.

According to the above described nebulizer system, it is possible to return the liquid that is not aerosolized into the bottle via the drain pipe disposed inside the heater device. In other words, unlike in conventional ones, the need for a drain tube that connects between the nebulizer adapter and the bottle, and is disposed outside the bottle is eliminated. Accordingly, it is possible to effectively prevent the nebulizer system from toppling when the drain tube is caught in a person's hand or thing. Further, since it is not necessary for the nebulizer adapter to include the drain tube, handling of work to connect a socket of the nebulizer adapter to a connecting section of the heater device can be quite easier.

Further, the heater device includes a liquid receiving section for storing the liquid that is not aerosolized in the nebulizer adapter, and it is preferable that the second end of the drain pipe is connected to the liquid receiving section. Further, the liquid receiving section is provided at the second end portion of the water conduit, and preferably includes a bottom and a side wall provided to erect from the bottom.

With such a configuration, it is possible to collect the liquid that is not aerosolized in the nebulizer adapter in the liquid receiving section, and to efficiently flow back the liquid to the bottle via the drain pipe inside the heater device.

Further, the second end portion of the water conduit passes through the bottom of the liquid receiving section, and it is preferable that the side wall is provided to erect from an outer circumferential edge of the bottom.

With such a configuration, a large space for collecting liquid in the liquid receiving section is available, and the liquid that is not aerosolized in the nebulizer adapter can be efficiently collected. Also, multiple drain pipes can be connected to the bottom of the liquid receiving section, and the liquid that is not aerosolized but stored in the liquid receiving section can be efficiently and quickly returned into the bottle.

The heater device includes a casing, and a cylindrical connecting section projecting from the casing, the nebulizer adapter being connected to the cylindrical connecting section. It is preferable that the nebulizer adapter includes a ring-shaped member having a sealing face capable of tight contact with an end surface of the connecting section.

With such a configuration, it is possible to effectively prevent humidified gas generated in the nebulizer adapter from leaking out of a connecting portion between the nebulizer adapter and the heater device.

Further, the water conduit and the drain pipe are preferably formed in a straight shape. With such a configuration, it is possible to efficiently clean throughout the inside of the water conduit and the drain pipe while visually checking the inside of the water conduit and the drain pipe.

Further, it is preferable that one side surface of the heater device and one side surface of the bottle are configured to be substantially flush with each other. With such a configuration, the nebulizer system can be installed such that the one side surface of the heater device and the one side surface of the bottle that are substantially flush with each other are in contact with a member with flat surface such as a wall. Accordingly, a stable installation position of the nebulizer system can be achieved while achieving space saving in the installation area.

Also disclosed is a heater device for a nebulizer system, the heater device being disposed between a bottle that has a bottle mouth on top thereof and stores liquid therein and a nebulizer adapter that sucks up the liquid in the bottle and generates fine aerosol from the sucked liquid. The heater device includes a water conduit, in which a first end portion thereof is connected to an end portion of the dip tube included in the bottle and a second end portion thereof is connected to a pipe-shaped aerosol-forming member included in the nebulizer adapter, a heating unit for heating the water conduit, and a drain pipe, in which a first end thereof is positioned to face inside the bottle mouth and a second end thereof is positioned to face inside the nebulizer adapter, the drain pipe flowing back the liquid that is not aerosolized in the nebulizer adapter into the bottle.

According to such a heater device, it is possible to return the liquid that is not aerosolized in the nebulizer adapter into the bottle via the drain pipe disposed inside the heater device. Consequently, the need for a conventional drain tube that connects between the nebulizer adapter and the bottle, and is disposed outside the bottle is eliminated. Accordingly, it is possible to effectively prevent the nebulizer system provided with the heater device from toppling when the drain tube is caught in a person's hand or thing.

### EFFECTS OF THE INVENTION

According to the present invention, it is possible to provide a nebulizer system that allows an easy connection of a bottle, a heater device, and a nebulizer adapter, while eliminating a drain tube that is exposed to the outside to get in the way, and a heater device preferably for use in the nebulizer system.

### BRIEF DESCRIPTION OF THE DRAWINGS

Fig. 1 is a perspective view of a nebulizer system according to one embodiment of the present invention.
Fig. 2 is a front view of a bottle included in the nebulizer system illustrated in Fig. 1.
Fig. 3 is a left side view of the bottle illustrated in Fig. 2.
Fig. 4 is a plane view of the bottle illustrated in Fig. 2.
Fig. 5 is a bottom view of the bottle illustrated in Fig. 2.
Fig. 6 is a cross-sectional view taken along a line A-A of Fig. 4.
Fig. 7 is a cross-sectional view taken along a line B-B of Fig. 4.
Fig. 8(a) is a schematic structural perspective view of a dip tube illustrated in Fig. 6, Fig. 8(b) is a schematic structural cross-sectional view of the dip tube, Fig. 8(c) is an enlarged plane view as seen from a direction of an arrow C in Fig. 8(b), and Fig. 8(d) is a cross-sectional view taken along a line D-D of Fig. 8(c).
Fig. 9(a) is a main part enlarged cross-sectional view of a diffuser section, and Fig. 9(b) is a main part enlarged front view of the diffuser section.
Fig. 10 is a main parts enlarged schematic structural cross-sectional view of the nebulizer system illustrated in Fig. 1.
Fig. 11 is a main part schematic structural view of a cross-section taken along a line E-E of Fig. 10.
Fig. 12 is a main part enlarged view illustrating a connection between a coupling section included in the heater device and a bottle mouth.
Fig. 13 is a main part enlarged view illustrating a connection between a socket included in the nebulizer adapter and a connecting section included in the heater device.
Fig. 14(a) is a plane view illustrating a variation of a liquid receiving section included in the heater device, Fig. 14(b) is a cross-sectional view taken along a line F-F of Fig. 14(a), and Fig. 14(c) is a structural view illustrating a state, in which a water conduit and a drain pipe are connected to the liquid receiving section, and a flow-path pipe is connected to the water conduit.
Fig. 15(a) is a plane view illustrating another variation of the liquid receiving section illustrated in Figs. 14(a) to (c), Fig. 15 (b) is a cross-sectional view taken along a line G-G of the Fig. 15(a), and Fig. 15(c) is a structural view illustrating a state, in which the water conduit and the drain pipe are connected to the liquid receiving section, and the flow-path pipe is connected to the water conduit.
Fig. 16 is a cross-sectional view illustrating a variation of the bottle illustrated in Fig. 2.
Fig. 17 is a schematic structural cross-sectional view of the nebulizer system, in which the heater device is not included.

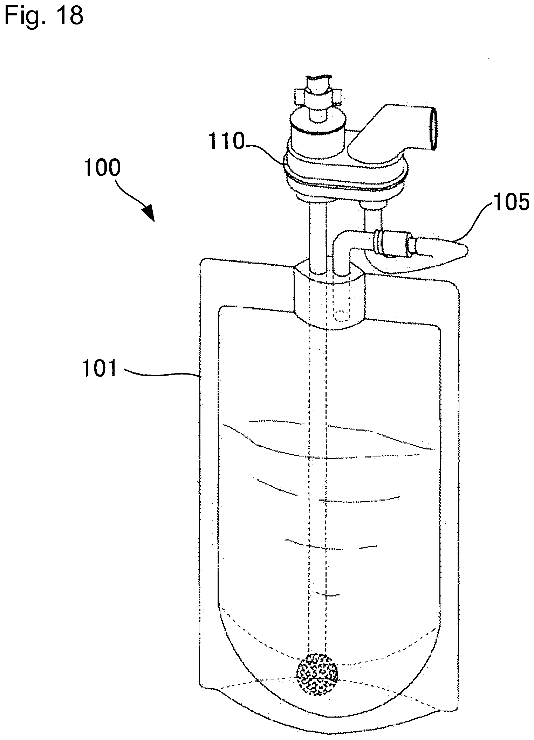

Fig. 18 is a schematic structural view illustrating a conventional nebulizer system.

### EMBODIMENTS OF THE INVENTION

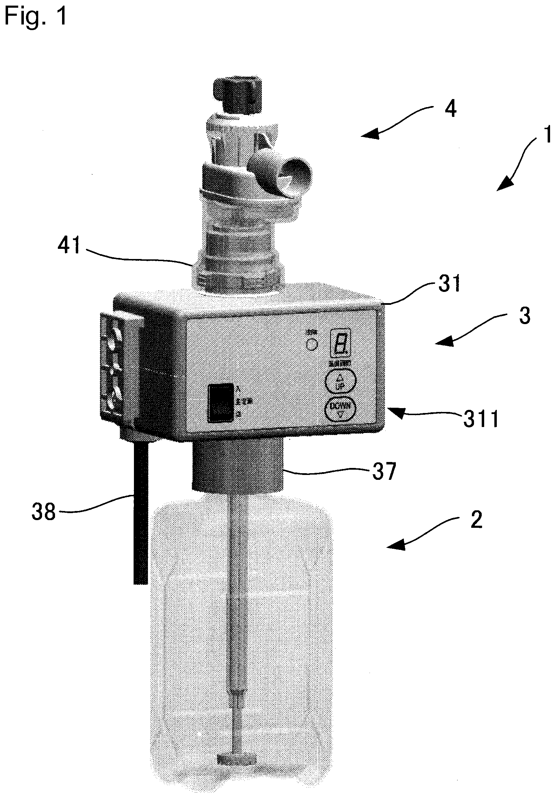

Hereinafter, a nebulizer system according to embodiments of the present invention will be described with reference to the accompanying drawings. Fig. 1 is a perspective view of the nebulizer system according to one embodiment of the present invention.

A nebulizer system 1 according to one embodiment of the present invention is a device for supplying humidified breathing gas, for example, and as illustrated in Fig. 1, includes a bottle 2, a heater device 3 that is disposed on top of the bottle 2, and a nebulizer adapter 4 that is disposed on top of the heater device 3.

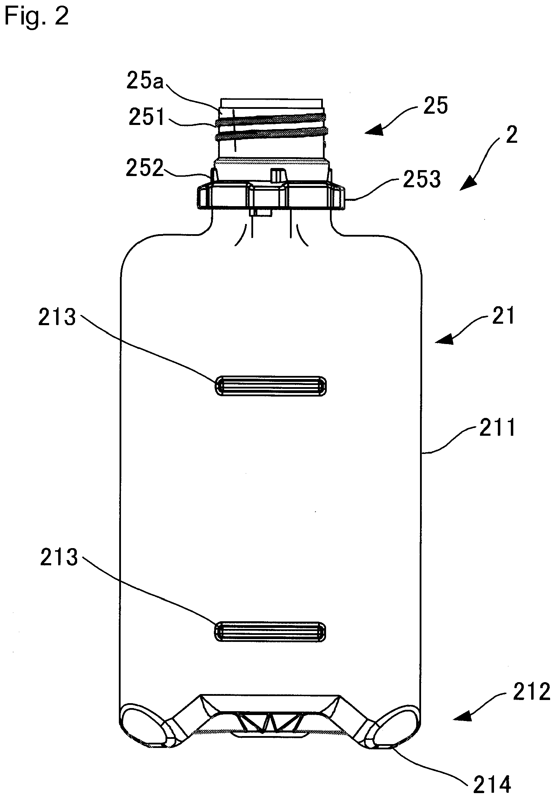

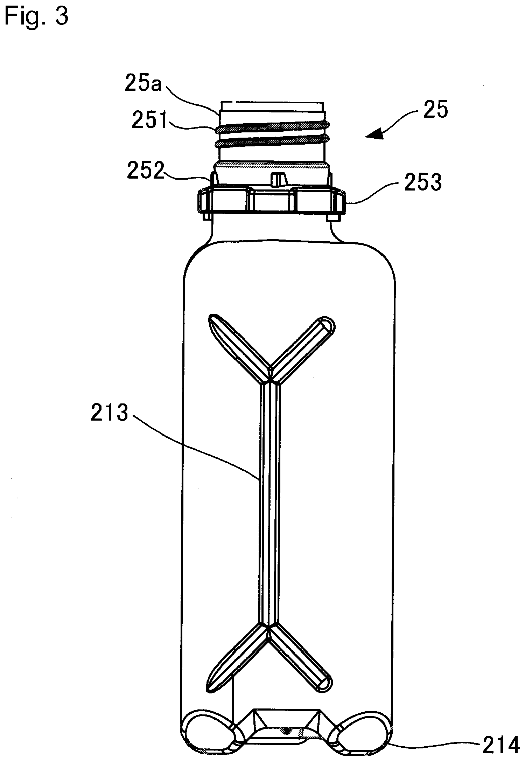

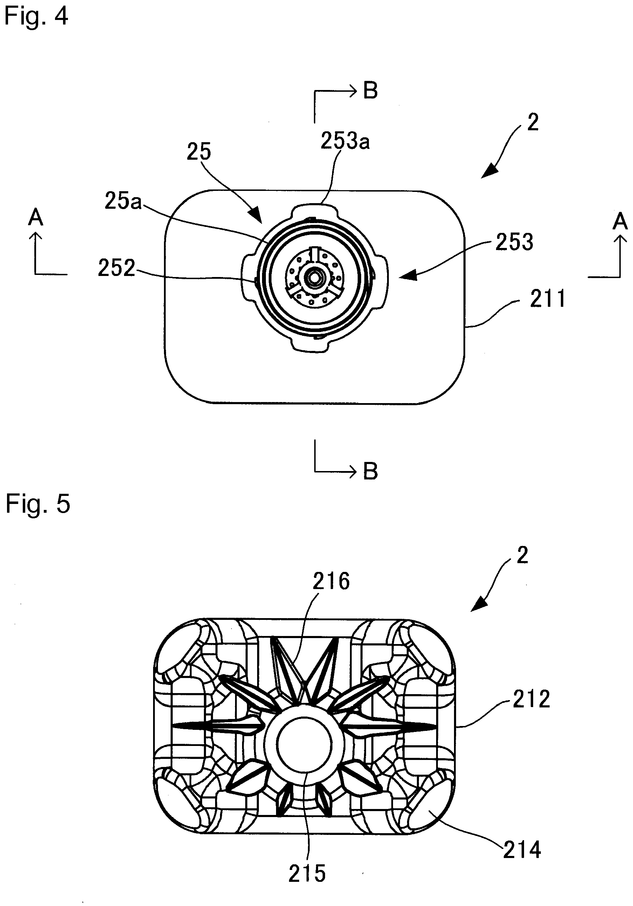

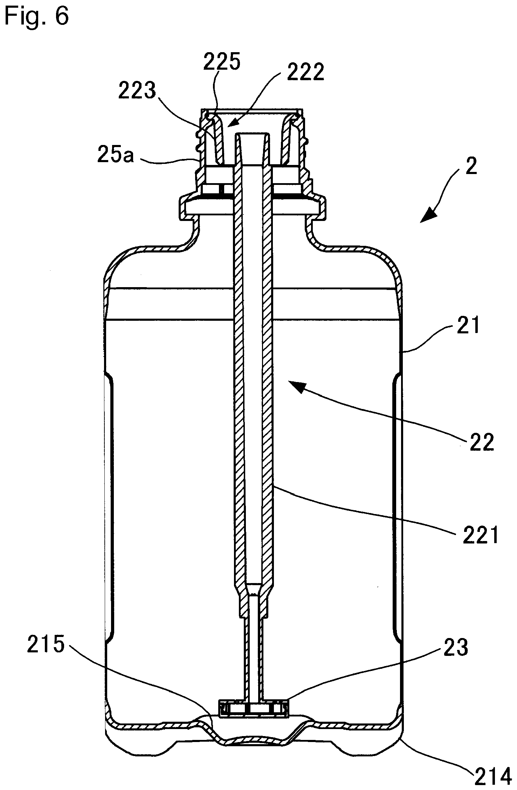

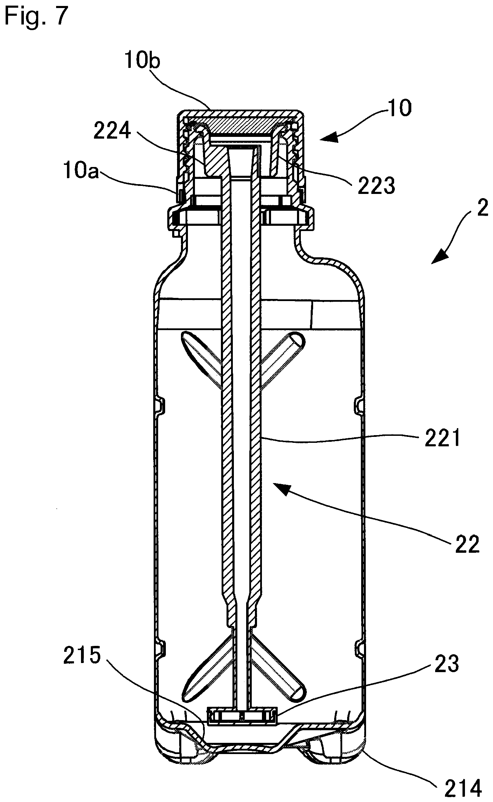

First, the bottle 2 will be described. The bottle 2 is a container that stores a solution in which medication is dissolved, or liquid such as sterile water, purified water, distilled water, and saline solution therein, and as illustrated in Figs. 2 to 7, a bottle main body 21 and a dip tube 22 are included. Fig. 2 is a front view of the bottle 2 and Fig. 3 is a left side view of the bottle 2. Fig. 4 is a plane view of the bottle 2 and Fig. 5 is a bottom view of the bottle 2. Fig. 6 is a cross-sectional view taken along a line A-A of Fig. 4, and Fig. 7 is a cross-sectional view taken along a line B-B of Fig. 4.

The bottle main body 21 includes a body 211 having a horizontal section formed in substantially rectangle shape, a bottom 212 that seals the lower end of the body 211, and a bottle mouth 25 that is provided on the upper end of the body 211 via its shoulder. On each side surface of the body 211, there are formed volume reduction ribs 213 that are constituted of a single linear recess or in combination of multiple linear recesses. These volume reduction ribs 213 are ribs for folding the side surfaces of the body 211 when the body 211 is crushed. The bottle 2 is configured to be reduced in volume by folding along the volume reduction ribs 213 formed at the side surfaces of the bottle main body 21 when the bottle main body 21 is crushed by pushing and pressing the front and back surfaces of the body 211, and then by folding the bottle 2 along the volume reduction ribs 213 formed at the front and back surfaces of the bottle 2.

Each of four corners of the bottom 212 of the bottle main body 21 is provided with a leg 214 for adding stability when the bottle 2 is placed. The leg 214 is formed to project outward from the bottom 212 downward in a vertical direction. In the central part of the bottom 212 of the bottle main body 21, there is formed a reservoir section 215 having a round plane view shape, in which the inner bottom surface of the bottle main body 21 is recessed downward in a vertical direction. The reservoir section 215 is formed such that the center thereof in a plane view coincides with the axial center of the bottle mouth 25. Further, pressure-proof ribs 216 are formed around the reservoir section 215 to encircle the reservoir section 215. The pressure-proof ribs 216 are formed to project outward from the bottom 212 of the bottle main body 21 downward in a vertical direction. It should be noted that the pressure-proof ribs 216 are provided to prevent deformation, such as expansion, of the bottom 212 of the bottle 2 due to the increase of inner pressure or the influence of heat when moist heat sterilization (e.g. autoclave sterilization) is performed after the bottle 2 is filled with the content liquid and capped or sealed.

The bottle mouth 25 is positioned on top of the bottle main body 21 and includes a thread 251, to which a cap 10 (refer to Fig. 7) having a thread structure can be fitted. The thread 251 is formed on the outer peripheral surface of a circumferential wall 25a of the bottle mouth 25. Further, in order to fit the separable cap 10 (tamper evident closure) that is a cap having a lower end portion being torn off to unseal the bottle, the circumferential wall 25a in the lower area of the thread 251 is formed to have a thick wall structure. On the outer peripheral portion of the thick wall structure, a multiple projections 252 is provided as stoppers. In other words, when the separable cap 10 is rotated to unseal the bottle, a ring portion 10a that is the lower portion of the separable cap 10 is caught on the projection and stays on the outer periphery of the thick wall structure, and only a cap main body 10b that is the upper portion of the separable cap 10 is to be separated. With such a configuration, the bottle 2 according to the present embodiment is sealable and permits sterilization process.

Also, the bottle mouth 25 includes an engagement 253 below the thick wall structure, on which the projections 252 as stoppers are formed. The engagement 253 is configured as a collection of a multiple projections 253a projecting outward from the circumferential wall 25a of the bottle mouth 25 in a horizontal direction. The engagement 253 is fitted with a coupling section 37 of the heater device 3.

As regards a material to form the bottle main body 21, a variety of materials can be used but for example, olefin polymers may be used. Typical examples of the olefin polymers are polyethylene and polypropylene-based polymers. Examples of the polyethylene include high density polyethylene (HDPE), low density polyethylene (LDPE), and blends thereof (HDPE/LDPE). Examples of the polypropylene-based polymers include polypropyne, random (or block) copolymers of polypropyne with other α-olefin such as ethylene, syndiotactic polypropylene, and blends thereof.

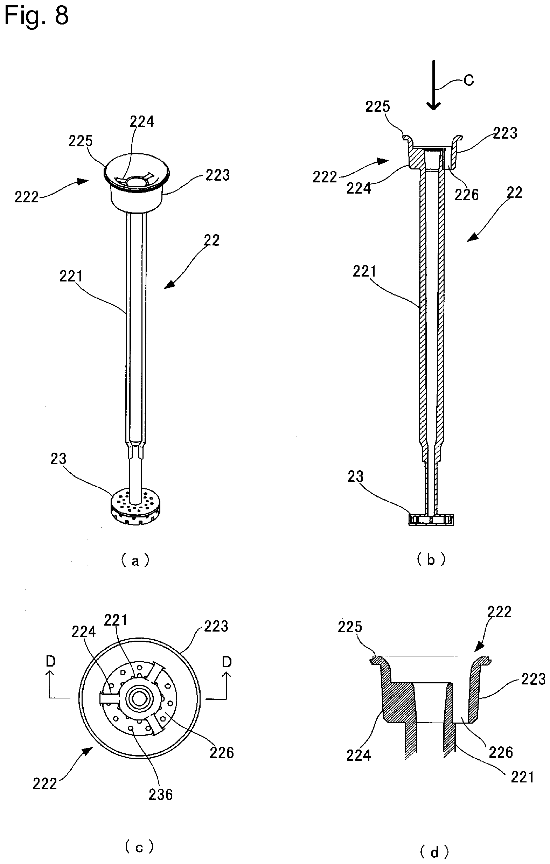

The dip tube 22 is a tubular body that is dipped into the liquid stored in the bottle main body 21, and includes a tube main body 221, a fixing section 222, and a diffuser section 23 as illustrated in Fig. 6 to Figs. 8(a), 8(b) and 8(c). Fig. 8(a) is a schematic structural perspective view of the dip tube 22, and Fig. 8(b) is a schematic structural cross-sectional view of the dip tube 22. Fig. 8(c) is an enlarged plane view as seen from a direction of an arrow C in Fig. 8(b), and Fig. 8(d) is a cross-sectional view taken along a line D-D of Fig. 8(c).

The tube main body 221 is constituted of a straight pipe, and a first end (upper end) thereof is connected by the fixing section 222 and a second end (lower end) thereof is connected by the diffuser section 23. The length of the tube main body 221 is set such that the second end is in the proximity of the bottom 212 of the bottle main body 21, when the first end is disposed inside the bottle mouth 25 (inside the area surrounded by the circumferential wall 25a).

The fixing section 222 is a component for positioning the dip tube 22 inside the bottle 2, and includes a cylindrical annulus 223 that is disposed on the inside of the bottle mouth 25, and a holding member 224 for holding and securing the tube main body 221 on the inside of the annulus 223. The annulus 223 is disposed in a position where the axial center thereof substantially coincides with the axial center of the bottle mouth 25. On the upper end portion of the annulus 223, a flange portion 225 is provided. The dip tube 22 can be positioned in the bottle main body 21 by having the flange portion 225 fitted into the upper end portion of the bottle mouth 25. Further, in order to form a space between the outer peripheral surface of the annulus 223 and the inner peripheral surface of the bottle mouth 25 (the inner peripheral surface of the circumferential wall 25a), the annulus 223 is disposed inside the bottle mouth 25 such that the outer peripheral surface of the annulus 223 and the inner peripheral surface of the bottle mouth 25 are spaced from each other.

The holding member 224 is a plate-shaped member extending in a vertical direction of the bottle mouth 25, and fixedly connects the inner peripheral surface of the annulus 223 with the outer peripheral surface of the tube main body 221. In the present embodiment, the tube main body 221 and the annulus 223 are coupled by three holding members 224 that are provided with a predetermined spacing along the circumferential direction of the inner peripheral surface of the annulus 223 (or the outer peripheral surface of the tube main body 221). With such a configuration, between each of the holding members 224 that is disposed between the inner peripheral surface of the annulus 223 and the outer peripheral surface of the tube main body 221, there is formed a clearance 226 communicating between the interior and exterior of the bottle main body 21 (a clearance 226 communicating between the both ends of the annulus 223). It should be noted that although the tube main body 221 and the annulus 223 are coupled by three holding members 224 in the present embodiment, the number of the holding members 224 is not particularly limited and, for example, the tube main body 221 and the annulus 223 may be coupled by a single holding member 224, two holding members 224, or four or more holding members 224.

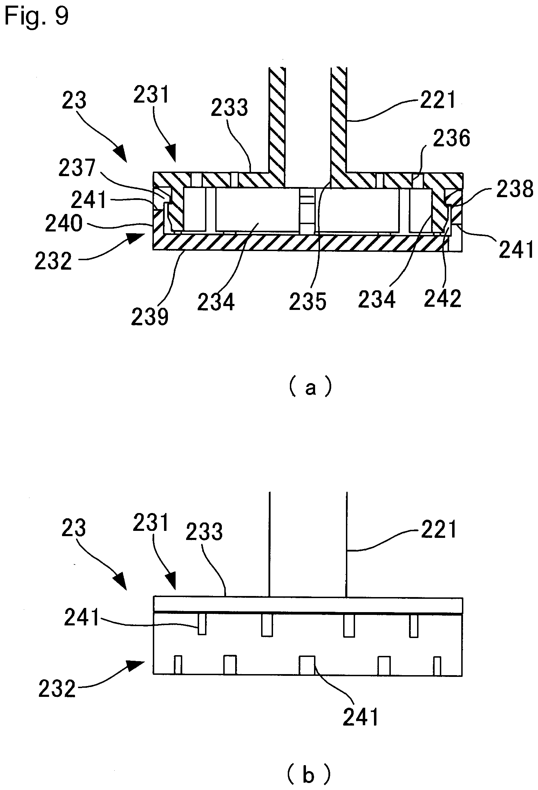

The diffuser section 23 is a component having a function of guiding the liquid stored in the bottle main body 21 into the tube main body 221, and is connected to the second end of the tube main body 221. As illustrated in Figs. 6 and 7, in a state where the dip tube 22 is positioned in the bottle main body 21, the diffuser section 23 is disposed directly over the reservoir section 215 that is formed on the bottom 212 of the bottle main body 21. The diffuser section 23 may be configured to be integral with the tube main body 221, or configured to be detachable from the tube main body 221. A configuration without the diffuser section 23 may also be employed. As illustrated in the main part enlarged view of Fig. 9, the diffuser section 23 includes an upper housing 231 and a lower housing 232. Fig. 9(a) is a main part enlarged cross-sectional view of the diffuser section 23, and Fig. 9(b) is a main part enlarged front view of the diffuser section 23.

The upper housing 231 includes a plate body 233 having a round plane view shape, in which a first surface thereof is connected to a lower end of the tube main body 221, and a multiple side walls 234 provided to erect on a second surface of the plate body 233. A slit is defined between each of the side walls 234. At the central portion of the plate body 233, there is formed an opening 235 passing through the plate body 233, and the tube main body 221 and the plate body 233 are connected so that the center of the opening 235 and the axial center of the tube main body 221 coincide with each other. Further, an opening diameter of the opening 235 and an inner diameter of the tube main body 221 are configured to have substantially the same size. Further, around the opening 235 that is formed at the central portion of the plate body 233, there is formed multiple fine first through holes 236 passing through the plate body 233. Each of the multiple first through holes 236 is disposed on concentric circles centered on the axial center of the tube main body 221. Further, the first through hole 236 is formed such that a hole diameter increases in size as the position where the first through hole 236 is formed is distanced from the opening 235. Each of the side walls 234 is disposed in the proximity of the circumferential edge of the plate body 233 with a predetermined spacing along the circumferential direction of the plate body 233. On the outer peripheral portion of the side wall 234, there is formed a fitting recess 238 that is engaged with a fitting projection 237 of the lower housing 232.

The lower housing 232 includes a plate-shaped lid 239 having a round plane view shape that covers the area surrounded by the side wall 234 of the upper housing 231, and a cylindrical circumferential wall 240 provided to erect from the circumferential edge of the lid 239. On an inner surface of the circumferential wall 240, there is formed the fitting projection 237 that is engaged with the fitting recess 238 formed on an outer peripheral surface of the side wall 234 of the upper housing 231. Further, on the circumferential wall 240, there is formed multiple second through holes 241 passing through the circumferential wall 240. Each of the second through holes 241 is formed with a predetermined spacing along the circumferential direction of the circumferential wall 240. Further, it is preferable that each of the second through holes 241 is formed in each of upper end and lower end portions of the circumferential wall 240.

Here, in a case where the lower housing 232 is installed in the upper housing 231, the circumferential wall 240 and the side wall 234 are formed so that the circumferential wall 240 of the lower housing 232 is placed outside of the side wall 234 of the upper housing 231, and so that a clearance 242 is formed between the inner peripheral surface of the circumferential wall 240 of the lower housing 232 and the outer peripheral surface of the side wall 234 of the upper housing 231. It should be noted that the lower housing 232 may be installed in the upper housing 231 such that the slit formed between each of the side walls 234 of the upper housing 231 and the second through holes 241 formed in the lower housing 232 will not coincide with each other. Alternatively, the lower housing 232 may be installed in the upper housing 231 such that a portion of the slit and a portion of the second through hole 241 will coincide with each other.

Further, although the circumferential wall 240 of the lower housing 232 is configured to be positioned outside the side wall 234 of the upper housing 231 in the present embodiment, the present invention is not particularly limited to such a configuration and, for example, the circumferential wall 240 of the lower housing 232 may be positioned inside the side wall 234 of the upper housing 231. In the case where such a configuration is employed, it is preferable that each of the slits extending along the axial center of the tube main body 221 is formed on the circumferential wall 240 of the lower housing 232 and the side wall 234 of the upper housing 231 is formed to have a cylindrical shape.

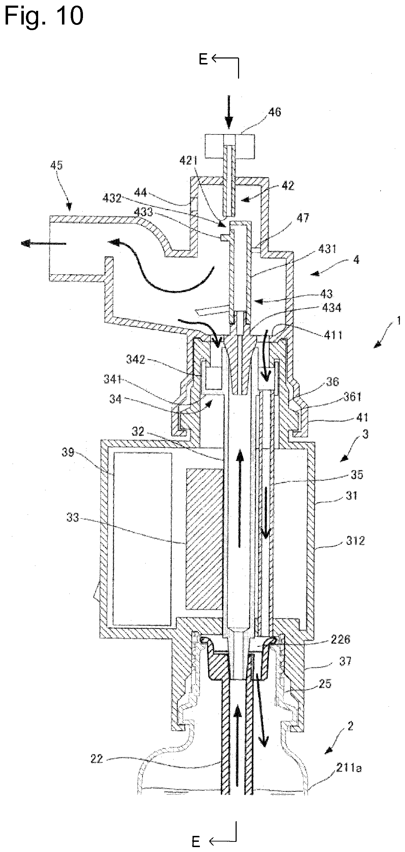

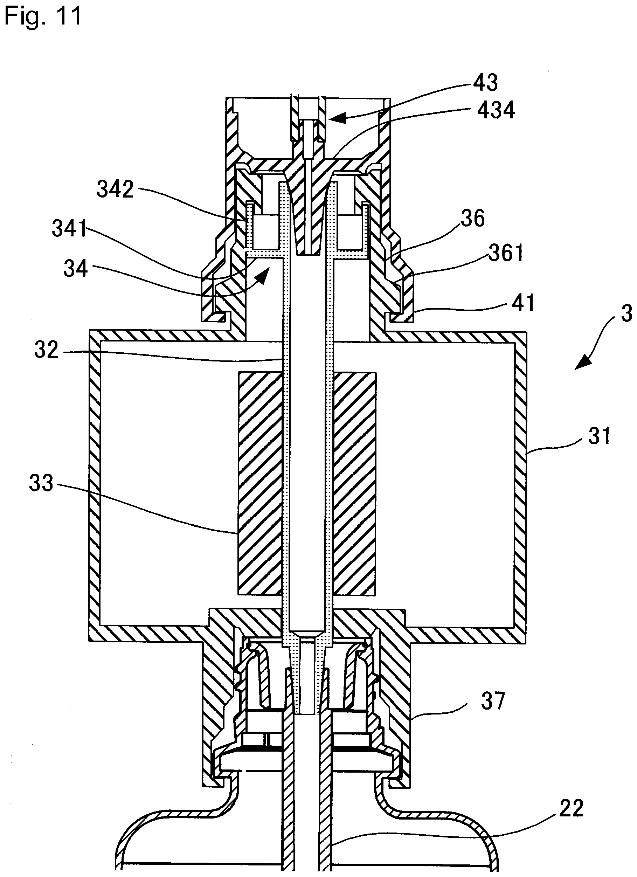

Next, the heater device 3 will be described. The heater device 3 is configured for connection to the bottle mouth 25, and includes a rectangular parallelepiped casing 31, a water conduit 32, a heating unit 33, a liquid receiving section 34, a drain pipe 35, a connecting section 36, the coupling section 37, and a power cord 38, as illustrated in Fig. 1, Fig. 10, and Fig. 11. Fig. 10 is a main part enlarged schematic structural cross-sectional view of the nebulizer system 1, and Fig. 11 is a main part schematic structural view of a cross-section taken along a line E-E of Fig. 10.

On the front of the casing 31, there is formed an operating unit 311 that is constituted of a switch for switching the power supply on or off, a setting button for setting the temperature of the heating unit 33, an indicator lamp for indicating the status of the operation of the heating unit 33, and the like. Further, in the present embodiment, in a state where the heater device 3 is connected to the bottle 2, one side surface 211a of the bottle 2 that faces the same direction as a back surface 312 of the casing 31 is configured to be substantially flush with the back surface 312 of the casing 31 (refer to Fig. 10). Herein, the back surface 312 of the casing 31 refers to one side surface opposite to the front of the casing where the operating unit 311 is formed. Further, inside the casing 31, there is disposed a control circuit 39 that controls driving of the heating unit 33 based upon input signals from the operating unit 311. The power cord 38 is connected to the control circuit 39 and the heating unit 33 via the bottom surface of the casing 31.

The water conduit 32 is a straight pipe member that is disposed inside the casing 31, a first end portion thereof is connected to communicate with the upper end portion of the dip tube 22 and a second end portion thereof is connected to communicate with a flow-path pipe 434 included in an under mentioned aerosol-forming member 43. The water conduit 32 includes metallic material such as stainless steel, or heat-resisting synthetic resin material.

The heating unit 33 is positioned inside the casing 31, is a component for heating the water conduit 32, and includes, for example, a resistance heating element. The heating unit 33 is configured to be able to surround the water conduit 32, and generates heat using power supply from the power cord 38 and heats the water conduit 32. By heating the water conduit 32, the liquid passing inside the water conduit 32 is heated.

The liquid receiving section 34 is positioned at the second end portion of the water conduit 32, and includes a disk-shaped bottom 341, and a side wall 342 provided to erect from the bottom 341. The liquid receiving section 34 in the present embodiment has a configuration, in which the second end portion of the water conduit 32 passes through the central portion of the bottom 341, and the side wall 342 is provided to erect from the outer circumferential edge of the bottom 341. In other words, the liquid receiving section 34 is defined by the outer peripheral surface at the second end portion of the water conduit 32, the side wall 342, and the bottom 341. The side wall 342 of the liquid receiving section 34 is tightly contact with the inner peripheral surface of the under mentioned cylindrical connecting section 36, and is configured so that all of the liquid that is not aerosolized in the nebulizer adapter 4 can be received and stored in the liquid receiving section 34.

The drain pipe 35 is a pipe member for flowing back the liquid that is not aerosolized in the nebulizer adapter 4 into the bottle 2. The drain pipe 35 is formed in a straight shape, and is positioned substantially in parallel with respect to the water conduit 32. Further, from the viewpoint of suppressing conduction and absorption of heat of the liquid in the water conduit 32 that is heated by the heating unit 33 into the drain pipe 35 and the liquid passing inside the drain pipe 35, it is preferable that the drain pipe 35 is positioned so as not to contact with the water conduit 32. The drain pipe 35 is formed of metallic material such as stainless steel, or synthetic resin material. A first end of the drain pipe 35 is positioned to face the clearance 226 of the bottle mouth 25. A second end of the drain pipe 35 is positioned to face inside the nebulizer adapter 4, and the second end is connected to the bottom 341 of the liquid receiving section 34. The liquid stored in the liquid receiving section 34 can be returned into the bottle 2 via the clearance 226 of the bottle mouth 25.

The connecting section 36 is a cylindrical port that projects from the upper portion of the casing 31, the nebulizer adapter 4 being connected to the cylindrical port. The second end portion and the liquid receiving section 34 of the above described water conduit 32 are positioned inside the connecting section 36. At the lower end portion (in the proximity of the connection with the casing 31) of the connecting section 36, there is formed a securing section 361 that has a similar configuration to that of the engagement 253 of the bottle mouth 25. A socket 41 of the nebulizer adapter 4 is engaged to the securing section 361.

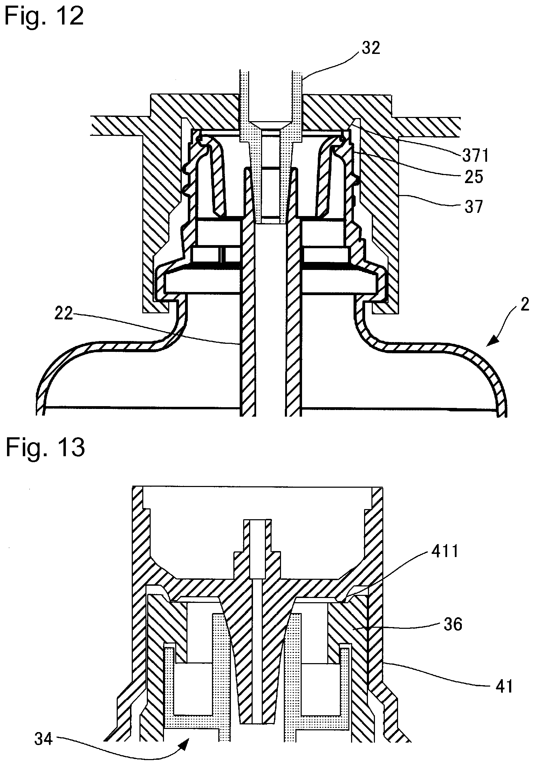

The coupling section 37 is a cylindrical member that projects from the lower portion of the casing 31, and is connected over the bottle mouth 25. The first end of the above described water conduit 32 is positioned inside the coupling section 37. Further, when the coupling section 37 is connected to the bottle mouth 25, in order to increase the air tightness therebetween, the coupling section 37 includes a sealing member 371 having a sealing face capable of tight contact with the end surface of the bottle mouth 25 (refer to the enlarged view illustrated in Fig. 12). From the viewpoint of increasing the air tightness between the sealing member 371 and the bottle mouth 25, it is preferable that each of them includes materials of varying hardness. For example, it is preferable that the sealing member 371 includes stainless steel, and the bottle mouth 25 (bottle 2) includes polypropylene.

Next, the nebulizer adapter 4 will be described. As illustrated in the perspective view of Fig. 1, and the cross-sectional views of Fig. 10 and Fig. 11, the nebulizer adapter 4 includes the socket 41, a nozzle member 42, the aerosol-forming member 43, an air suction hole 44, and a guide section 45.

The socket 41 is a cylindrical member that is connected over the connecting section 36 of the heater device 3. At the inner peripheral surface of the socket 41, there is provided a ring-shaped member 411 having a sealing face capable of tight contact with the end surface of the connecting section 36 included in the heater device 3 (refer to the enlarged view illustrated in Fig. 13). From the viewpoint of increasing the air tightness between the ring-shaped member 411 and the connecting section 36, it is preferable that each of them includes materials of varying hardness. For example, it is preferable that the ring-shaped member 411 includes polycarbonate, and the connecting section 36 includes polyacetal.

The nozzle member 42 is a tubular body that injects oxygen gas from an orifice 421 that is provided at the lower end thereof, and at the upper end thereof, includes a gas source connecting section 46 that is connected to a gas source (not shown).

The aerosol-forming member 43 is a member that is disposed in a direction of the gas injection from the nozzle member 42, the member sucks up sterile water via the dip tube 22 and the water conduit 32 by a stream of the injected gas, and generates fine aerosol from the sucked sterile water. Specifically, the aerosol-forming member 43 includes a pipe body 431 having an upper end being closed, a through hole 432 that is formed in the proximity of the upper end of the pipe body 431, and is in communication with an internal passage, a projecting member 433 that is provided on the surface of the pipe body 431 below the through hole 432 and projects outward, and a flow-path pipe 434 that is connected to communicate with the lower end of the pipe body 431. The flow-path pipe 434 is a tubular body that is connected to communicate with the water conduit 32 positioned inside the heater device 3 in the state where the socket 41 is connected to the connecting section 36 of the heater device 3. The flow-path pipe 434 is fixed inside the socket 41 by a rod-shaped fixing member 47 that connects between the outer peripheral surface of the flow-path pipe 434 and the inner peripheral surface of the cylindrical socket 41. The air suction hole 44 is a hole, through which air is sucked in association with the gas injection by the nozzle member 42. The guide section 45 is a cylindrical member that is provided on a side surface of the socket 41, and guides a mixed gas of oxygen containing aerosol and air to the outside.

Next, operation of the nebulizer system 1 with such a configuration will be described with reference to Fig. 10. When the nozzle member 42 injects oxygen gas supplied from a gas source (not shown) toward the projecting member 433, by a stream of the injected gas, a negative pressure is developed around the through hole 432, and the sterile water (liquid) contained in the bottle 2 flows out of the through hole 432 via the diffuser section 23, the dip tube 22, the water conduit 32, the flow-path pipe 434, and the pipe body 431. The sterile water (liquid) thus discharged creates fine aerosol by the action of oxygen gas blasted toward the projecting member 433. The aerosol is mixed with the oxygen gas, further mixed with the air being sucked from the air suction hole 44, and guided to the outside via the guide section 45 for supplying to a patient.

In the sterile water (liquid) that is discharged from the through hole 432 via the diffuser section 23, the dip tube 22, the water conduit 32, the flow-path pipe 434, and the pipe body 431, droplets that a not aerosolized will fall down inside the socket 41, and will be collected in the liquid receiving section 34 included in the heater device 3. Then the droplets will pass through the drain pipe 35 that is connected to the bottom 341 of the liquid receiving section 34, and will return into the bottle main body 21 via the clearance 226 formed in the bottle mouth 25. Also, a bottom of the guide section 45 is configured to be sloped toward the socket 41. The droplets stored in the bottom of the guide section 45 will also fall down inside the socket 41, will be collected in the liquid receiving section 34, and will return into the bottle main body 21 via the drain pipe 35.

The nebulizer system 1 according to the present embodiment allows the liquid that is not aerosolized by the nebulizer adapter 4 to be returned into the bottle 2 via the drain pipe 35 that is disposed inside the heater device 3. In other words, unlike conventional ones, the need for a drain tube that connects between the nebulizer adapter 4 and the bottle 2, and is disposed outside the bottle 2 is eliminated. Accordingly, it is possible to effectively prevent the nebulizer system from toppling when the drain tube is caught in a person's hand or thing. Further, since it is not necessary for the nebulizer adapter 4 to include the drain tube, setting work to connect the socket 41 of the nebulizer adapter 4 to the connecting section 36 of the heater device 3 can be quite easier. In addition, unlike conventional ones, as a result that the need for the drain tube that connects between the nebulizer adapter 4 and the bottle 2, and is disposed outside the bottle 2 is eliminated, it is possible to effectively prevent the liquid in the bottle 2 from being contaminated due to contamination of the drain tube.

Further, the heater device 3 includes the liquid receiving section 34 for storing the liquid that is not aerosolized in the nebulizer adapter 4, and has a configuration, in which the second end of the drain pipe 35 is connected to the liquid receiving section 34. Therefore, it is possible to collect the liquid that is not aerosolized in the nebulizer adapter 4 in the liquid receiving section 34, and efficiently flow back the liquid into the bottle 2 via the drain pipe 35.

Further, the liquid receiving section 34 is configured such that the second end portion of the water conduit 32 passes through the bottom 341 of the liquid receiving section 34, and the side wall 342 is provided to erect from the outer circumferential edge of the bottom 341. Therefore, a large space for collecting liquid in the liquid receiving section 34 is available, and the liquid that is not aerosolized in the nebulizer adapter 4 can be efficiently collected. Also, two or more drain pipes 35 can be connected to the bottom 341 of the liquid receiving section 34, and thus the liquid that is not aerosolized and stored in the liquid receiving section 34 can be efficiently and quickly returned into the bottle 2.

The heater device 3 includes the cylindrical connecting section 36 projecting from the casing 31, the nebulizer adapter 4 being connected to the cylindrical connecting section 36. Since the nebulizer adapter 4 includes the ring-shaped member 411 having a sealing face capable of tight contact with the end surface of the connecting section 36, it is possible to effectively prevent humidified gas generated in the nebulizer adapter 4 from leaking out of the connecting portion between the nebulizer adapter 4 and the heater device 3.

Further, since the water conduit 32 and the drain pipe 35 included in the heater device 3 are formed in a straight shape, it is possible to efficiently clean throughout the inside of the water conduit 32 and the drain pipe 35 while visually checking the both insides of the water conduit 32 and the drain pipe 35.

Further, since the one side surface 312 of the heater device 3 and the one side surface 211a of the bottle 2 are configured to be substantially flush with each other, the nebulizer system 1 can be installed such that the one side surface 312 of the heater device 3 and the one side surface 211a of the bottle 2 that are substantially flush with each other are in contact with a member with flat surface such as a wall. Accordingly, space saving in the installation area and a stabile installation position of the nebulizer system 1 can be achieved.

Also, the outer peripheral surface at the lower end portion of the annulus 223 of the dip tube 22 included in the bottle 2 is disposed to be spaced from the inner peripheral surface of the bottle mouth 25. Therefore, the droplets that pass through the drain pipe 35 of the heater device 3 and return into the bottle 2 via the clearance 226 will flow down along the inner peripheral surface of the annulus 223 and will fall down from the lower end of the annulus 223 toward the liquid in the bottle 2. With this configuration, it is possible to effectively prevent the droplets returned from the nebulizer adapter 4 from adhering to adjacent areas of the inner peripheral surface of the bottle mouth 25. In a case where droplets stay at adjacent areas of the inner peripheral surface of the bottle mouth 25, these droplets are less prone to falling (flowing) downward, which might cause the growth of various germs. However, with such a configuration, it is possible to effectively prevent various germs from growing in adjacent areas of the inner peripheral surface of the bottle mouth 25.

Further, the holding member 224 included in the fixing section 222 of the dip tube 22 is a plate-shaped member extending in a vertical direction of the bottle mouth 25, and is configured to connect the inner peripheral surface of the annulus 223 with the outer peripheral surface of the tube main body 221. With such a configuration, without reducing the flow area of the clearance 226 (i.e. the area of the horizontal section of the clearance 226), through which droplets flow, it is possible to reinforce the connection between the annulus 223 and the tube main body 221.

Although the embodiment of the nebulizer system 1 according to the present invention has been described above, the specific configuration is not limited to the above described embodiment. For example, in the above described embodiment, although the drain pipe 35 that is disposed inside the heater device 3 is configured to have a straight shape, and is disposed substantially parallel with respect to the water conduit 32 that is surrounded by the heating unit 33, the drain pipe 35 may be formed in a shape extending along the inner peripheral surface of the casing 31 of the heater device 3. With such a configuration, since the drain pipe 35 can be disposed in a position away from the heating unit 33, it is possible to effectively reduce the liquid that passes through the drain pipe 35 and is guided into the bottle 2 from being heated by the heating unit 33. Additionally, it is also possible to effectively prevent the heat in the heating unit 33 from being taken by the liquid passing inside the drain pipe 35, and as a result, it is possible that the heating unit 33 efficiently heats the liquid passing inside the water conduit 32.

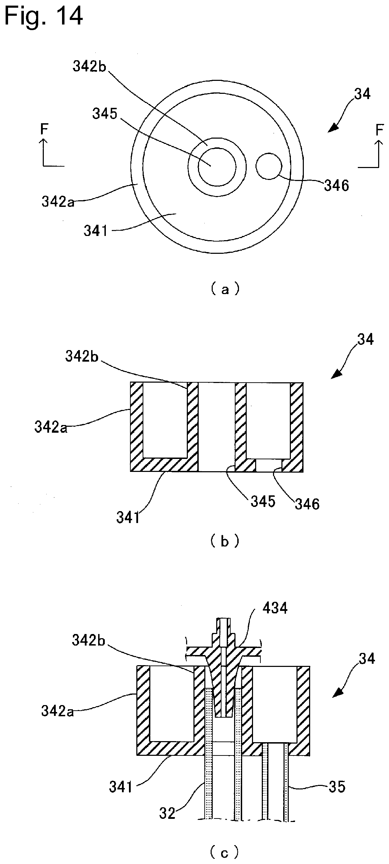

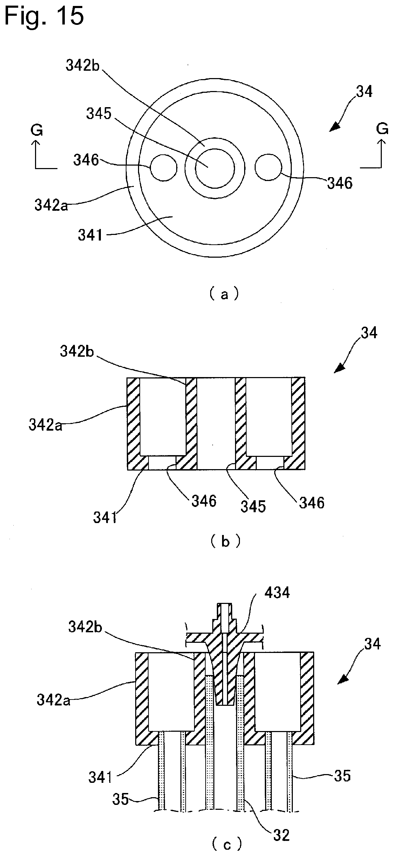

The configuration of the liquid receiving section 34 included in the heater device 3 is not particularly limited to the above described configuration. For example, as illustrated in Fig. 14, the liquid receiving section 34 may be configured such that a through hole 345 is formed at the substantially central portion of the disk-shaped bottom 341, an outer side wall 342a is provided to erect from the outer circumferential edge of the bottom 341, and an inner side wall 342b is provided around the through hole 345. It should be noted that a connecting hole 346 to which the drain pipe 35 is connected is formed in a predetermined area of the bottom 341 positioned between the inner side wall 342b and the outer side wall 342a. In the case where such a configuration is employed, the liquid receiving section 34 is installed by inserting the second end portion of the water conduit 32 into the through hole 345. Here again, Fig. 14(a) is a plane view of the liquid receiving section 34 and Fig. 14(b) is a cross-sectional view taken along a line F-F of Fig. 14(a). Further, Fig. 14(c) is a structural view illustrating a state in which the water conduit 32 and the drain pipe 35 are connected to the liquid receiving section 34, and a flow-path pipe 434 is connected to the water conduit 32. The configuration of the liquid receiving section 34 is illustrated in Fig. 15, in which two or more drain pipes 35 are connected. In Fig. 15, on the bottom 341 of the liquid receiving section 34, two connecting holes 346 are formed, to which the drain pipes 35 are connected so that the through hole 345 is positioned therebetween. Fig. 15(a) is a plane view of the liquid receiving section 34 and Fig. 15(b) is a cross-sectional view taken along a line G-G of Fig. 15(a). Further, Fig. 15(c) is a structural view illustrating a state in which the water conduit 32 and the drain pipe 35 are connected to the liquid receiving section 34, and the flow-path pipe 434 is connected to the water conduit 32.

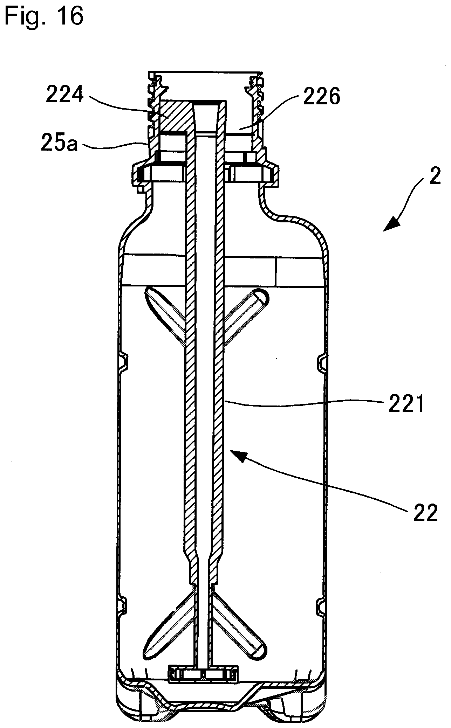

Also, in a state where the clearance 226 communicating between the interior and exterior of the bottle main body 21 via bottle mouth 25 is provided, the upper end of the dip tube 22 that is disposed inside the bottle 2 may be disposed inside the circumferential wall 25a of the bottle mouth 25. For example, the dip tube 22 may have such a configuration as illustrated in Fig. 16. The dip tube 22 illustrated in Fig. 16 is defined by the tube main body 221 and the holding member 224. The holding member 224 illustrated in Fig. 16 has the function of the fixing section 222, and is formed to connect the inner peripheral surface of the circumferential wall 25a of the bottle mouth 25 with the outer peripheral surface of the tube main body 221. In the case where such a configuration is employed, it is preferable that the holding member 224 includes a plate-shaped member extending in a vertical direction of the bottle mouth 25. With such a configuration, it is possible to effectively guide the droplets that are returned from the inside of the nebulizer adapter 4 to the bottle 2 via the clearance 226 into the tube main body 221 so that the droplets flow down along the outer surface of the tube main body 221. As a result of this, it is possible to effectively prevent the droplets returned to the bottle 2 from being stored in adjacent areas of the inner peripheral surface of the bottle mouth 25.

Further, the bottle 2 in the above described embodiment has a configuration in which the annulus 223 included in the fixing section 222 has the flange portion 225 fitted into the upper end portion of the bottle mouth 25. However, a configuration may also be employed in which, for example, without forming the flange portion 225, the annulus 223 is pushed into the inner side of the bottle mouth 25, and the outer peripheral surface of the annulus 223 is fitted by the inner peripheral surface of the bottle mouth 25. In the case where such a configuration is employed, it is preferable to reduce the diameter of the lower end portion of the annulus 223, such as for example by providing a tapered portion, so that the outer peripheral surface at the lower end portion of the annulus 223 is spaced from the inner peripheral surface of the bottle mouth 25. With the configuration in which the diameter of the lower end portion of the annulus 223 is reduced, it is possible to prevent the droplets returned from the nebulizer adapter 4 from adhering to adjacent areas of the inner peripheral surface of the bottle mouth 25.

Further, in the above described embodiment, the holding member 224 that fixedly connects the inner peripheral surface of the annulus 223 with the outer peripheral surface of the tube main body 221 includes a plate-shaped member extending in a vertical direction of the bottle mouth 25. However, the present invention is not particularly limited to such a configuration, and for example, the holding member 224 may include a rod-shaped member, and the annulus 223 and the tube main body 221 may be connected with each other.

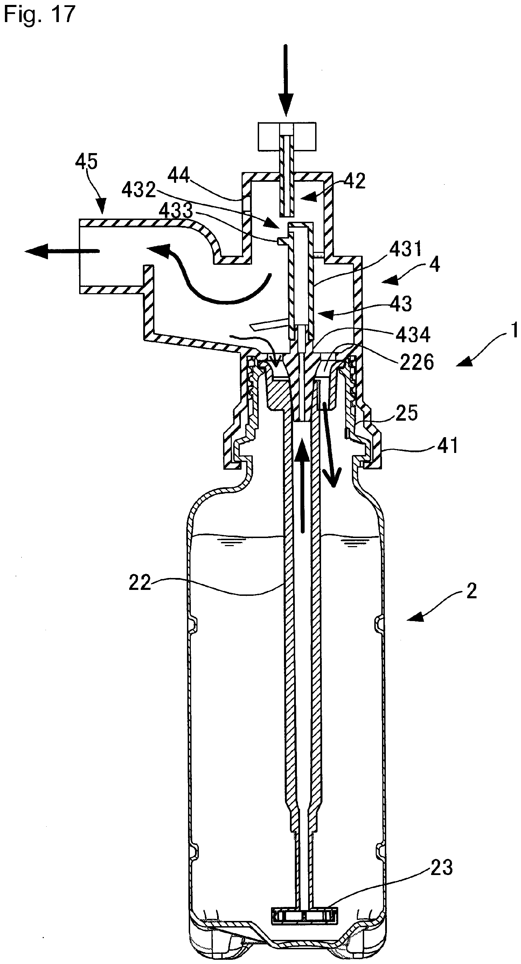

It should be noted that the nebulizer system 1 according to the above described embodiment can be used without the heater device 3. In the case where the heater device 3 is not mounted, the socket 41 of the nebulizer adapter 4 is connected to the bottle mouth 25 directly. Due to the above connection, the flow-path pipe 434 is connected to communicate with the dip tube 22. Usage of the nebulizer system 1 in such a state will be described with reference to Fig. 17. When the nozzle member 42 injects oxygen gas supplied from a gas source (not shown) toward the projecting member 433, by a stream of the injected gas, a negative pressure is developed around the through hole 432, the sterile water (liquid) contained in the bottle 2 is flowing out of the through hole 432 via the diffuser section 23, the dip tube 22, the flow-path pipe 434, and the pipe body 431. The sterile water (liquid) thus discharged creates fine aerosol by the action of oxygen gas blasted toward the projecting member 433. The aerosol is mixed with the oxygen gas, further mixed with the air being sucked from the air suction hole 44, and guided to the outside via the guide section 45 to supply to a patient.

In the sterile water (liquid) that is discharged from the through hole 432 via the diffuser section 23, the dip tube 22, the flow-path pipe 434, and the pipe body 431, droplets that are not aerosolized will fall down inside the socket 41, and will return into the bottle 2 via the clearance 226 formed in the bottle mouth 25. The droplets stored in the bottom of the guide section 45 will also fall down inside the socket 41, and will return into the bottle 2 via the clearance 226 formed in the bottle mouth 25.

### DESCRIPTION OF REFERENCE SIGNS

- 1: nebulizer system
- 2: bottle
- 21: bottle main body
- 22: dip tube
- 25: bottle mouth
- 226: clearance
- 3: heater device
- 32: water conduit
- 33: heating unit
- 34: liquid receiving section
- 35: drain pipe
- 4: nebulizer adapter
- 42: nozzle member
- 421: orifice
- 43: aerosol-forming member

## Claims

1. A nebulizer system (1) comprising:
a bottle (2) that has a bottle mouth (25) on top thereof and stores liquid therein;
a heater device (3) that is connectable to the bottle mouth (25); and
a nebulizer adapter (4) that is connectable to the heater device (3), wherein
the bottle (2) includes a dip tube (22) that is dipped into the liquid stored therein, an upper end portion of the dip tube (22) being disposed inside a circumferential wall of the bottle mouth (25) with a clearance (226) communicating between an interior and an exterior of the bottle via the bottle mouth,
the nebulizer adapter (4) includes a nozzle member (42) that has an orifice (421) for injecting gas, and an aerosol-forming member (43) in a corresponding position with the orifice (421), the aerosol-forming member (43) sucking up the liquid in the bottle (2) via the dip tube (22) and heater device (3) by a stream of gas injected from the orifice (421), and generating fine aerosol from the sucked liquid,
the heater device (3) includes:
a water conduit (32) in which a first end portion thereof is connected to an upper end portion of the dip tube (22) and a second end portion thereof is connected to the aerosol-forming member (43),
a heating unit (33) for heating the water conduit (32), and
a drain pipe (35) in which a first end thereof is positioned to face the clearance (226), the drain pipe (35) flowing back the liquid that is not aerosolized in the nebulizer adapter (4) into the bottle (2) via a second end portion thereof,
**characterized in that** the dip tube (22) comprises:
a tube main body (221); and
a fixing section (222) for positioning the dip tube (22) inside the bottle (2), the fixing section (222) being connected to a first end of the tube main body (221), the fixing section (222) comprising:
a cylindrical annulus (223) that is disposed on the inside of the bottle mouth (25), the clearance (226) being provided between an inner circumferential surface of the annulus (223) and an outer circumferential surface of the tube main body (221); and
a holding member (224) for holding and securing the tube main body (221) on the inside of the annulus (223).

2. The nebulizer system (1) according to claim 1, wherein
the heater device (3) includes a liquid receiving section (34) for receiving the liquid that is not aerosolized in the nebulizer adapter (4), and the second end of the drain pipe (35) is connected to the liquid receiving section(34).

3. The nebulizer system (1) according to any one of claims 1 and 2, wherein the liquid receiving section (34) is provided at the second end portion of the water conduit (32), and includes a bottom (341), and a side wall (342) provided to erect from the bottom (341).

4. The nebulizer system (1) according to claim 3, wherein
the second end portion of the water conduit (32) passes through the bottom of the liquid receiving section(34), and
the side wall (342) is provided to erect from an outer circumferential edge of the bottom (341).

5. The nebulizer system (1) according to any one of claims 2 to 4, wherein
the heater device (3) includes a casing (31), and a cylindrical connecting section (36) projecting from the casing (31), the nebulizer adapter (4) being connected the cylindrical connecting section (36),
the nebulizer adapter (4) includes a ring-shaped member (411) having a sealing face capable of contact with an end surface of the connecting section (36).

6. The nebulizer system (1) according to any one of claims 1 to 5, wherein the water conduit (32) and the drain pipe (35) are formed in a straight shape.

7. The nebulizer system (1) according to any one of claims 1 to 6, wherein one side surface of the heater device (3) and one side surface of the bottle (2) are configured to be flush with each other.

## Patentansprüche

1. Zerstäubersystem (1) mit:
einer Flasche (2), die an ihrer Oberseite einen Flaschenhals (25) aufweist, und in der Flüssigkeit bevorratet ist;
einer Heizeinrichtung (3), die mit dem Flaschenhals (25) verbindbar ist; und
einem Zerstäuberadapter (4), der mit der Heizeinrichtung (3) verbindbar ist, wobei
die Flasche (2) ein Steigrohr (22) aufweist, das in die darin bevorratete Flüssigkeit eingetaucht ist, wobei ein oberer Endabschnitt des Steigrohrs (22) innerhalb einer Umfangswand des Flaschenhalses (25) mit einem Freiraum (226) angeordnet ist, der eine Verbindung zwischen einem Inneren und einem Äußeren der Flasche über den Flaschenhals herstellt,
der Zerstäuberadapter (4) ein Mundstückelement (42), das eine Mündung (421) zum Injizieren von Gas aufweist, und ein aerosolbildendes Element (43) in einer zu der Mündung (421) korrespondierenden Position umfasst, wobei das aerosolbildende Element (43) die Flüssigkeit in der Flasche (2) über das Steigrohr (22) und die Heizeinrichtung (3) mittels eines von der Mündung (421) injizierten Gasstroms ansaugt und ein feines Aerosol aus der angesaugten Flüssigkeit erzeugt,
die Heizeinrichtung (3) aufweist:
eine Wasserleitung (32), bei welcher ein erster Endabschnitt davon mit einem oberen Endabschnitt des Steigrohrs (22) verbunden ist und ein zweiter Endabschnitt davon mit dem aerosolbildenden Element (43) verbunden ist,
eine Heizeinheit (33) zum Heizen der Wasserleitung (32), und
ein Abflussrohr (35), bei welchem ein erstes Ende davon dem Freiraum (226) zugewandt positioniert ist, wobei das Abflussrohr (35) über einen zweiten Endabschnitt davon die in dem Zerstäuberadapter (4) nicht aerosolierte Flüssigkeit in die Flasche (2) zurückleitet,
**dadurch gekennzeichnet, dass** das Steigrohr (22) umfasst:
einen Rohrhauptkörper (221); und
einen Fixierabschnitt (222) zum Positionieren des Steigrohrs (22) im Inneren der Flasche (2),
wobei der Fixierabschnitt (222) mit einem ersten Ende des Rohrhauptkörpers (221) verbunden ist und der Fixierabschnitt (222) umfasst:
ein zylinderförmiges Ringstück (223), das auf der Innenseite des Flaschenhalses (25) angeordnet ist, wobei der Freiraum (226) zwischen einer Innenumfangsfläche des Ringstücks (223) und einer Außenumfangsfläche des Rohrhauptkörpers (221) angeordnet ist, und
ein Halteelement (224) zum Halten und Befestigen des Rohrhauptkörpers (221) an der Innenseite des Ringstücks (223).

2. Zerstäubersystem (1) nach Anspruch 1, wobei
die Heizeinrichtung (3) eine Flüssigkeitsaufnahmesektion (34) zum Aufnehmen der in dem Zerstäuberadapter (4) nicht aerosolierten Flüssigkeit umfasst und das zweite Ende des Abflussrohrs (35) mit der Flüssigkeitsaufnahmesektion (34) verbunden ist.

3. Zerstäubersystem (1) nach einem der Ansprüche 1 und 2, wobei die Flüssigkeitsaufnahmesektion (34) an dem zweiten Endabschnitt der Wasserleitung (32) vorgesehen ist und einen Boden (341) und eine an dem Boden (341) aufrecht angesetzte Seitenwand (342) aufweist.

4. Zerstäubersystem (1) nach Anspruch 3, wobei
der zweite Endabschnitt der Wasserleitung (32) durch den Boden des Flüssigkeitsaufnahmeabschnitts (34) hindurch geht und
die Seitenwand (342) an einer Außenumfangskante des Bodens (341) aufrecht angesetzt ist.

5. Zerstäubersystem (1) nach einem der Ansprüche 2 bis 4, wobei
die Heizeinrichtung (3) ein Gehäuse (31) und eine von dem Gehäuse (31) vorstehende zylindrische Verbindungssektion (36) aufweist, wobei der Zerstäuberadapter (4) mit der zylindrischen Verbindungssektion (36) verbunden ist,
der Zerstäuberadapter (4) ein ringförmiges Element (411) umfasst, das eine Dichtungsfläche aufweist, die mit einer Endfläche der Verbindungssektion (36) kontaktierbar ist.

6. Zerstäubersystem (1) nach einem der Ansprüche 1 bis 5, wobei die Wasserleitung (32) und das Abflussrohr (35) in gerader Form ausgebildet sind.

7. Zerstäubersystem (1) nach einem der Ansprüche 1 bis 6, wobei eine Seitenfläche der Heizeinrichtung (3) und eine Seitenfläche der Flasche (2) miteinander bündig eingerichtet sind.

## Revendications

1. Système nébuliseur (1) comprenant :
une bouteille (2) qui comporte un goulot de bouteille (25) en son sommet et dans laquelle est stocké un liquide ;
un dispositif de chauffage (3) qui peut être raccordé au goulot de bouteille (25) ; et
un adaptateur de nébuliseur (4) qui peut être raccordé au dispositif de chauffage (3),
dans lequel :
la bouteille (2) comporte un tube plongeur (22) qui est plongé dans le liquide qui y est stocké, une partie d'extrémité supérieure du tube plongeur (22) étant disposée à l'intérieur d'une paroi circonférentielle du goulot de bouteille (25), un jeu (226) communicant entre l'intérieur et l'extérieur de la bouteille par l'intermédiaire du goulot de bouteille,
l'adaptateur de nébuliseur (4) comporte un élément formant buse (42) qui présente un orifice (421) destiné à injecter du gaz, et un élément formant aérosol (43) à une position correspondant à l'orifice (421), l'élément formant aérosol (43) aspirant le liquide de la bouteille (2) par l'intermédiaire du tube plongeur (22) et du dispositif de chauffage (3) au moyen d'un flux gazeux injecté par l'orifice (421), et produisant de fines gouttelettes d'aérosol à partir du liquide aspiré,
le dispositif de chauffage (3) comporte :
un conduit d'eau (32) dont une première partie d'extrémité est raccordée à une partie d'extrémité supérieure du tube plongeur (22) et dont une seconde partie d'extrémité est raccordée à l'élément formant aérosol (43) ;
une unité de chauffage (33) destinée à chauffer le conduit d'eau (32) ; et
un tuyau de vidange (35) dont une première extrémité est positionnée en regard du jeu (226), le tuyau de vidange (35) faisant refluer le liquide qui n'est pas transformé en aérosol dans l'adaptateur de nébuliseur (4), vers la bouteille (2) par l'intermédiaire d'une seconde partie d'extrémité de celui-ci,
**caractérisé en ce que** le tube plongeur (22) comprend :
un corps principal de tube (221) ; et
une section de fixation (222) destinée à positionner le tube plongeur (22) à l'intérieur de la bouteille (2),
la section de fixation (222) étant raccordée à une première extrémité du corps principal de tube (221), la section de fixation (222) comprenant :
un anneau cylindrique (223) qui est disposé sur l'intérieur du goulot de bouteille (25), le jeu (226) étant ménagé entre une surface circonférentielle intérieure de l'anneau (223) et une surface circonférentielle extérieure du corps principal de tube (221) ; et
un élément de maintien (224) destiné à maintenir et à assujettir le corps principal de tube (221) sur l'intérieur de l'anneau (223).

2. Système nébuliseur (1) selon la revendication 1, dans lequel :
le dispositif de chauffage (3) comporte une section de réception de liquide (34) destinée à recevoir le liquide qui n'est pas transformé en aérosol dans l'adaptateur de nébuliseur (4), et la seconde extrémité du tuyau de vidange (35) est raccordée à la section de réception de liquide (34).

3. Système nébuliseur (1) selon l'une quelconque des revendications 1 et 2, dans lequel la section de réception de liquide (34) est placée à la seconde partie d'extrémité du conduit d'eau (32), et présente un fond (341) et une paroi latérale (342) destinée à s'ériger à partir du fond (341).

4. Système nébuliseur (1) selon la revendication 3, dans lequel :
la seconde partie d'extrémité du conduit d'eau (32) passe à travers le fond de la section de réception de liquide (34), et
la paroi latérale (342) est destinée à s'ériger à partir d'un bord circonférentiel extérieur du fond (341).

5. Système nébuliseur (1) selon l'une quelconque des revendications 2 à 4, dans lequel :
le dispositif de chauffage (3) comporte une enveloppe (31) et une section cylindrique de raccordement (36) faisant saillie à partir de l'enveloppe (31), l'adaptateur de nébuliseur (4) étant raccordé à la section cylindrique de raccordement (36),
l'adaptateur de nébuliseur (4) comporte un élément annulaire (411) présentant une face d'étanchéité apte à entrer en contact avec une surface d'extrémité de la section de raccordement (36).

6. Système nébuliseur (1) selon l'une quelconque des revendications 1 à 5, dans lequel le conduit d'eau (32) et le tuyau de vidange (35) sont réalisés selon une forme droite.

7. Système nébuliseur (1) selon l'une quelconque des revendications 1 à 6, dans lequel une surface latérale du dispositif de chauffage (3) et une surface latérale de la bouteille (2) sont conçues pour être dans l'alignement l'une de l'autre.
